(19) Europäisches Patentamt
European Patent Office
Office européen des brevets

(11) Numéro de publication: 0 116 182
B2

(12) # NOUVEAU FASCICULE DE BREVET EUROPÉEN

(45) Date de publication du nouveau fascicule du brevet: **27.12.89**

(51) Int. Cl.⁴: **C 07 C 83/10**

(21) Numéro de dépôt: **83201719.8**

(22) Date de dépôt: **05.12.83**

(54) **Procédé de préparation d'acide p-butoxyphénylacéthydroxamique à l'état finement divisé.**

(30) Priorité: **13.12.82 LU 84530**

(43) Date de publication de la demande:
**22.06.84 Bulletin 84/34**

(45) Mention de la délivrance du brevet:
**03.09.86 Bulletin 86/36**

(45) Mention de la decision concernant l'opposition:
**27.12.89 Bulletin 89/52**

(84) Etats contractants désignés:
**AT BE CH DE FR GB IT LI NL**

(56) Documents cité:
**DE-C-1 280 254**
**DE-C-17 684 06**

**CHEMICAL ABSTRACTS, vol. 96, no. 18, 3 mai 1982, réf. no. 149082t, page 415, Columbus Ohio (US)**
**Lehrbuch der pharmazeutischen Technologie, R. Vogt, M. Borschein, Verlag Chemie, 1975, Seiten 143-144**

(73) Titulaire: **CONTINENTAL PHARMA, INC., Avenue Louise, 135, B-1050 Bruxelles (BE)**

(72) Inventeur: **Franz, Michel, rue de Grand Bigard 121 Berchem- Sainte- Agathe, B-1080 Bruxelles (BE)**
Inventeur: **Vincze, Andras, Avenue de Broqueville 235 Woluwé- Saint- Lambert, B-1200 Bruxelles (BE)**
Inventeur: **Lambelin, Georges, rue Cervantès 31 Forest, B-1190 Bruxelles (BE)**
Inventeur: **Polat, Alain, Chemin du Pré- Longuet, CH- 1213 Onex- Genève (CH)**

(74) Mandataire: **Callewaert, Jean, Bureau Gevers S.A. rue de Livourne 7 bte 1, B-1050 Bruxelles (BE)**

EP 0 116 182 B2

LIBER, STOCKHOLM 1989

**Description**

La présente invention est relative à un procédé de préparation d'acide p-butoxyphénylacéthydroxamique à l'état finement divisé.

L'acide p-butoxyphénylacéthydroxamique, connu sous le nom générique "Bufexamac", est une molécule à activité antiinflammatoire reconnue qui répond à la formule suivante:

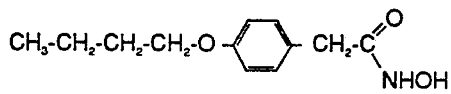

Cette molécule présente une très faible solubilité dans l'eau et ses sels sont peu stables.

D'une façon générale il est intéressant de disposer, dans le cas d'un principe médicamenteux de faible solubilité dans l'eau, d'une variété de matière première correspondant à un produit finement divisé encore appelé "micronisé".

D'une part, l'équation de Noyes et Whitney qui régit le phénomène de dissolution, que doit connaître le médicament avant d'être absorbé, indique que la vitesse de dissolution est proportionnelle à la surface des particules en contact avec les milieux physiologiques.

L'utilisation d'une taille particulaire réduite offrant une grande surface est donc un élément bénéfique lors de la préparation de formes à usage oral, comme les comprimés, les gélules et les capsules molles ou de formes à usage topique ou rectal.

D'autre part, dans le cas de produits peu hydrosolubles, la réalisation de plusieurs formes galéniques sous forme de suspensions à partir d'une substance finement divisée, à échelle microscopique, est avantageuse à différents points de vue: tolérance locale améliorée dans le cas d'injections ou de collyres et réalisation technologiqué des formes galéniques plus aisées, notamment dans le cas de produits injectables.

L'obtention d'un produit antiinflammatoire à l'état finement divisé, comme le bufexamac, permet notamment la réalisation de collyres et de formes injectables intra-articulaires dans un domaine thérapeutique où les dérivés de la cortisone sont largement employés; ceci constitue un progrès car les dérivés cortisoniques présentent des effets secondaires préjudiciables au niveau de certaines fonctions hormonales, des processus de cicatrisation et de la défense immunologique.

Les méthodes physiques de réduction de la taille des particules (broyage à l'aide du moulin colloïdal, microniseur à air comprimé) ne conduisent pas à des résultats satisfaisants dans le cas du bufexamac: les cristaux de départ ne se laissent pas fractionner et les produits obtenus présentent un important pourcentage de décomposition.

Des techniques de microcristallisation ont été testées, sans donner de résultats favorables: critallisation dirigée, action des ultrasons, technique de dilution et de reprécipitation, solvatation et désolvation successives.

D'autre part, il est connu du document Pharm. Weckbl. Sci. Ed. 1982 4(1), 5 - 11, A. Ludwig et al: "The influence of the cristallisation on the properties of a hydrophobic drug" - de recristaller la phénacétine à partir de solutions saturées chaudes dans l'éthanol ou dans la glycérine, en les versant dans une solution aqueuse d'une substance tensioactive (polysorbate 80). Par ce procédé connu le diamètre des particules obtenues varie en fonction de la nature du solvant utilisée et la concentration en substance tensioactive. C'est ainsi que le diamètre des partiules des lots les plus fins est encore d'environ 7 micromètres.

Un but essentiel de l'invention est de présenter un procédé de préparation d'acide p-butoxyphénylacéthydroxamique à l'état finement divisé, notamment sous forme de particules microscopiques, permettant de remédier aux inconvénients des procédés connus cités ci-dessus.

A cet effet, le procédé suivant l'invention consiste à dissoudre l'acide p-butoxyphénylacéthydroxamique dans une solution basique d'agent salifiant à une température comprise entre 40 et 75°C, avantageusement entre 50 et 60°C, et de préférence à 55°C ± 3°C et à le précipiter ensuite dans une solution acide à température ambiante en présence d'une substance tensio-active non-ionique dont la balance hydrolipophile est comprise entre 10 et 18.

D'autres détails et particularités de l'invention ressortiront de la description donnée ci-après, à titre d'exemples non limitatifs, de quelques formes de réalisation du procédé suivant l'invention.

Après de multiples essais et de façon surprenante, il a été constaté, suivant l'invention, que l'introduction d'une solution aqueuse chaude d'un sel de bufexamac dans une solution aqueuse acide contenant un tensio-actif permet d'obtenir une suspension de cristaux de taille très réduite, chimiquement purs et physiquement stables.

La salification du bufexamac est de préférence réalisée par une base asséz forte et pharmaceutiquement acceptable; l'emploi de sels de métaux alcalins est recommandé. Préférentiellement, l'hydroxyde de sodium sera utilisé.

Des résultats très satisfaisant sont obtenus lorsque la solution aqueuse d'agent salifiant est préalablement chauffée à une température se situant entre 40 et 75°C, ou mieux entre 50 et 60°C et idéalement à 55°C ±

3°C.

La quantité d'agent salifiant employée sera avantageusement calculée en léger excès afin de dissoudre le bufexamac dans le temps le plus court.

L'acide utilisé pour neutraliser le sel formé extemporanément peut être organique ou inorganique; il devra être compatible avec l'administration à l'homme ou à l'animal.

Parmi les acides qui peuvent être employés, les acides tartrique, aspartique, citrique, phosphorique, sulfurique et chlorhydrique sont à mentionner.

La quantité d'acide introduite est calculée de façon à neutraliser la base et à libérer le bufexamac finement divisé.

Pour préparer et préserver les particules de bufexamac dans une forme finement divisée, il est nécessaire de former un film interfacial entre les particules et le milieu ambiant.

Ce film doit être de préférence réalisé à l'aide d'un tensioactif non-ionique, seul compatible notamment avec l'administration par injection.

Un tensioactif est une substance qui possède un caractère mixte par suite de l'existence en son sein de groupements fonctionnels hydrophiles et lipophiles.

Des liaisons doivent pouvoir être établies entre les groupements fonctionnels du tensioactif et le bufexamac.

La valeur de la balance hydro-lipophile est la caractéristique fondamentale d'un tensioactif nonionique; elle représente la capacité du tensioactif à disperser un produit dans son milieu.

Ce pouvoir est influencé par la polarité des groupements fonctionnels (par exemple hydroxyle alcoolique, oxyde d'éthylène, acide gras), leur nature, leur structure, le degré de condensation etc..

La valeur de la balance hydrolipophile (ou hydrolipophilic balance = HLB) représente le rapport des tendances hydrophiles (H) et lipophiles (L) de la molécule.

Artificiellement, la valeur 10 à été attribuée aux tensioactifs où les deux types de groupement sont en équilibre.

Après plusieurs essais, il est apparu qu'une valeur de HLB comprise entre 10 et 18 était souhaitable pour la préparation du bufexamac finement divisé avec un très bon rendement.

Pour éviter tout phénomène de dégradation, la préparation du sel et la neutralisation par précipitation dans la solution acide contenant le tensioactif doit se dérouler avantageusement dans un délai de quelques minutes.

Le produit finement divisé peut être isolé ou préparé dans le milieu final de la formulation sans isolement de la matière première.

L'analyse granulométrique du bufexamac obtenu a été réalisée grâce à un appareillage Coulter Counter; la taille particulaire correspondait bien à un produit finement divisé qui restait stable au cours de la conservation, comme l'indique le tableau suivant.

**Analyse granulométrique**

1) Médiane Nombre *
2) Médiane Poids **
3) Taille maximum rencontrée

| | | Lot 795/016 A | Lot 795/016 B | Lot 796/018 A | Lot 796/018 B |
|---|---|---|---|---|---|
| au départ | 1) | 1,88 | | 1,77 | |
| | 2) | 2,76 | | 1,98 | |
| | 3) | 19,32 | | 19,32 | |
| après 8 mois | 1) | 1,85 | 1,84 | 1,87 | 1,88 |
| | 2) | 2,32 | 2,75 | 2,73 | 2,63 |
| | 3) | 19,32 | 19,32 | 19,32 | 12,17 |
| après 36 mois | 1) | 2,09 | 2,13 | 1,88 | 2,09 |
| | 2) | 2,93 | 3,28 | 2,90 | 3,21 |
| | 3) | 19,32 | 15,33 | 19,32 | 15,33 |

* Médiane Nombre: 50 % des particules (en nombre) ont un diamètre inférieur ou égal au chiffre mentionné.
** Médiane Poids: 50 % des particules (en poids ou volume) ont un diamètre inférieur ou égal au chiffre mentionné.

Le tableau indique en microns les mesures réalisées sur deux lots de suspension conservés à température ordinaire (colonnes A) et à température de 35°C (colonnes B).

La stabilité chimique du produit finement divisé a également été démontrée au cours du temps, notamment à l'aide des méthodes chromatographiques.

Les exemples qui suivent sont donnés pour illustrer l'invention sans la limiter en aucune façon.

**Exemple 1**

Composition:

| | | |
|---|---|---|
| | Bufexamac | 2 000 g |
| a. | Hydroxyde sodique | 360 g |
| | Eau déminéralisée | 14 300 ml |
| b. | Acide citrique cristallisé | 1 070 g |
| | Tween 80 ® | 360 g |
| | Eau déminéralisée | 36 000 ml |

**Mode opératoire:**

1. Préparer une solution d'hydroxyde sodique avec les composants a. Porter cette solution à une température comprise entre 55 et 58°C. Y dissoudre le bufexamac.
2. Préparer une solution acidé de tensioactif avec les composants b.
3. Verser la solution chaude (55° - 58°) du sel sodique de bufexamac dans la solution acide qui est maintenue sous agitation à température ambiante.
4. Le précipité obtenu est filtré sur papier et lavé à l'aide d'eau distillée jusqu'à obtention d'un filtrat neutre.
5. Sécher.

**Exemple 2**

Composition:

| | | |
|---|---|---|
| | Bufexamac | 100 g |
| a. | Hydroxyde potassique | 25,2 g |
| | Eau déminéralisée | 715 ml |
| b. | Acide chlorhydrique | 17,7 g |
| | Tween 60 ® | 20 g |
| | Eau déminéralisée | 1800 ml |

**Mode opératoire:**

1. Porter à une température de 55° - 58°C une solution d'hydroxyde sodique avec les produits a.
2. Ajouter le bufexamac.
3. Agiter jusqu'à dissolution complète.
4. Préparer à température ambiante une solution acidé de tensioactif avec les produits b.
5. Verser la solution alcaline de bufexamac mainténue à 55° - 58°C dans la solution acide.
6. Le bufexamac obtenu est filtré sur papier et ensuite lavé avec de l'eau déminéralisée jusqu'à complète neutralité du filtrat.
7. Sécher.

Les produits préparés selon les exemples 1 et 2 peuvent être employés comme matière première dans la réalisation de formes orales, comme les comprimés, les gélules et les capsules molles, de formes à usage topique ou rectal.

Dans ces formulations, les excipients habituels peuvent être utilisés.

**Exemple 3**

Composition:

| | | |
|---|---|---|
| | Bufexamac | 20,6 g |
| a. | Hydroxyde sodique | 3,725 g |
| | Eau bidistillée | 250 ml |
| b. | Acide chlorhydrique 1 N | 97 ml |
| | Tween 80 ® | 4 g |
| | Eau bidistillée | 200 ml |
| c. | Carboxyméthylcellulose (CMC) | 10 g |
| | Agent conservateur | q.s. |
| | NaCl | 3 g |
| | Eau bidistillée q.s. ad | 1000 ml |

**Mode opératoire:**

1. Préparer une solution sodique avec les composants a. Filtrer cette solution stérilement sur filtre 0,22 μ.
2. Préparer une solution d'acide chlorhydrique et de Tween 80 avec les composants b. Filtrer cette solution stérilément sur filtre 0,22 μ.

3. Préparer une solution avec les composants c. Filtrer sur verre fritté n° 2. Stériliser à 120°C durant 20 minutes.
4. Porter la solution 1 à 55°C et y dissoudre le bufexamac préalablement tamisé.
5. Verser la solution 4 dans la solution 2 sous forte agitation.
6. A l'aide d'une solution d'hydroxyde sodique diluée ajuster le pH à une valeur de 6,5 ± 0,5.
7. Ajouter à la suspension la solution 3.
8. Distribuer en ampoules dans des conditions aseptiques.

**Exemple 4**

Composition:

| | |
|---|---|
| Bufexamac | 41,2 g |
| a. Hydroxyde sodique | 7,45 g |
| Eau bidistillée | 500 ml |
| b. Acide chlorhydrique 1 N | 194 ml |
| Tween 20 ® | 8,24 g |
| Eau bidistillée | 400 ml |
| c. Carboxyméthylcellulose (CMC) | 20 g |
| Alcool benzylique | 20 g |
| NaCl | 6 g |
| Eau bidistillée q.s. ad. | 2000 ml |

**Mode opératoire:**

1. Préparer à l'aide des composants a. une solution d'hydroxyde sodique. Filtrer cette solution stérilement sur filtre 0,22 μ.
2. Préparer à l'aide des composants b. une solution d'acide chlorhydrique et de Tween 20. Filtrer cette solution stérilement sur filtre 0,22 μ.
3. Dissoudre la CMC et le NaCl dans l'eau bidistillée renseignée en c. Filtrer sur verre fritté n° 2. Stériliser à 120°C durant 20 minutes. Ajouter ensuite l'alcool benzylique préalablement filtré.
4. Porter la solution 1 à 55°C et y dissoudre le bufexamac tamisé.
5. Verser la solution 4 dans la solution 2.
6. Porter le pH à 8.5 ± 0.5 à l'aide d'une solution d'hydroxyde sodique diluée.
7. Ajouter la solution 3 à la suspension obtenue en 6.
8. Distribuer stérilement en flacons-collyre stériles.

Dans ces exemples, le mot "Tween" désigne des esters de sorbitane polyoxyéthylénés commercialisés par la société "Atlas Chemical".

Il est bien entendu que l'invention n'est pas limitée aux formes de réalisation décrites et que bien des variantes peuvent être envisagées sans sortir du cadre du présent brevet.

C'est ainsi, par exemple, que l'on peut introduire directement un sel de bufexamac dans la solution acide contenant le tensioactif.

## Revendications

1. Procédé de préparation d'acide p-butoxyphénylacéthydroxamique micronisé, caractérisé en ce qu'il consiste à dissoudre de l'acide p-butoxyphénylacéthyldroxamique dans une solution basique d'agent salifiant à une température comprise entre 40 et 75°C, avantageusement entre 50 et 60°C, et de préférence à 55°C ± 3°C et à le précipiter ensuite dans une solution acide à température ambiante en présence d'une substance tensio-active non-ionique dont la balance hydrolipophile est comprise entre 10 et 18.

2. Procédé suivant la revendication 1, caractérisé en ce qu'il consiste à ajouter la solution d'agent salifiant à la solution acide en maintenant cette dernière sous agitation.

3. Procédé suivant l'une ou l'autre des revendications 1 ou 2, caractérisé en ce qu'on utilise une solution d'agent salifiant contenant une base relativement forte.

4. Procédé suivant la revendication 3, caractérisé en ce qu'il consiste à dissoudre l'acide p-butoxyphénylacéthydroxamique dans une solution d'une base de métal alcalin.

5. Procédé suivant l'une quelconque des revendications 1 à 4, caractérisé en ce qu'on utilise une solution d'agent salifiant en léger excès par rapport à la quantité d'acide p-butoxyphénylacéthydroxamique à y dissoudre.

6. Procédé suivant l'une quelconque des revendications 1 à 5, caractérisé en ce qu'on utilise, pour la préparation de

l'acide p-butoxyphénylacéthydroxamique, une solution d'un acide choisi dans le groupe formé par l'acide tartrique, aspartique, citrique, phosphorique, sulfurique et chlorhydrique.

7. Procédé suivant l'une quelconque des revendications 1 à 6, caractérisé en ce qu'il consiste à précipiter l'acide p-butoxyphénylacéthydroxamique par addition d'une solution de sel sodique dans une solution d'acide chlorhydrique placée à température ambiante et contenant un ester de sorbitane polyoxyéthyléné.

8. Procédé suivant l'une quelconque des revendications 1 à 7, caractérisé en ce qu'il consiste à précipiter l'acide p-butoxyphénylacéthydroxamique en présence d'excipients appropriés sans isolement de l'acide p-butoxyphénylacéthydroxamique précipité.

**Patentansprüche**

1. Verfahren zur Herstellung von mikronisierter p-Butoxyphenylacethydroxamsäure, dadurch gekennzeichnet, daß man die p-Butoxyphenylacethydroxamsäure in einer basischen Lösung eines Salzbildners bei einer Temperatur zwischen 40 und 75°C, vorteilhaft zwischen 50 und 60°C und vorzugsweise bei 55°C ± 3°C löst und dann in einer sauren Lösung bei Umgebungstemperatur in Gegenwart eines nichtionischen Tensids ausfällt, dessen hydrophillipophiles Gleichgewicht zwischen 10 und 18 liegt.

2. Verfahren nach Anspruch 1, dadurch gekennzeichnet, daß man die Lösung des Salzbildners zu der sauren Lösung hinzugibt und dabei die letztere in Bewegung hält.

3. Verfahren nach einem der Ansprüche 1 oder 2, dadurch gekennzeichnet, daß man eine Lösung eines Salzbildners verwendet, der eine relativ starke Base enthält.

4. Verfahren nach Anspruch 3, dadurch gekennzeichnet, daß man die p-Butoxyphenylacethydroxamsäure in einer Lösung einer Alkalimetallbase löst.

5. Verfahren nach einem der Ansprüche 1 bis 4, dadurch gekennzeichnet, daß man die Lösung eines Salzbildners im leichten Oberschuß im Verhältnis zur Menge der darin zu lösenden p-Butoxyphenylacethydroxamsäure einsetzt.

6. Verfahren nach einem der Ansprüche 1 bis 5, dadurch gekennzeichnet, daß man zur Herstellung der p-Butoxyphenylacethydroxamsäure eine Lösung einer Säure verwendet, die aus der Weinsäure, Asparaginsäure, Zitronensäure, Phosphorsäure, Schwefelsäure und Salzsäure umfassenden Gruppe ausgewählt ist.

7. Verfahren nach einem der Ansprüche 1 bis 6, dadurch gekennzeichnet, daß man die p-Butoxyphenylacethydroxamsäure durch Zugabe einer Natriumsalzlösung zu einer Salzsäurelösung ausfällt, die sich bei Umgebungstemperatur befindet und einen polyoxyethylenierten Sorbitanester enthält.

8. Verfahren nach einem beliebigen der Ansprüche 1 bis 7, dadurch gekennzeichnet, daß man die p-Butoxyphenylacethydroxamsäure in Gegenwart geeigneter Trägerstoffe ausfällt, ohne die ausgefällte p-Butoxyphenylacethydroxamsäure zu isolieren.

**Claims**

1. A method for the preparation of micronised p-butoxyphenylacetylhydroxamic acid, characterised in that it comprises dissolving p-butoxyphenylacetylhydroxamic acid in a basic solution of salifying agent at a temperature of between 40 and 75°C, advantageously between 50 and 60°C and, preferably, at 55°C ± 3°C, and then precipitating it in an acid solution at ambient temperature in the presence of a non-ionic surface-active agent, the hydrolipophilic balance of which is between 10 and 18.

2. A method according to claim 1, characterised in that it comprises adding the solution of salifying agent to the acid solution, while the latter is maintained under agitation.

3. A method according to either claim 1 or claim 2, characterised in that a solution of salifying agent is used containing a relatively strong base.

4. A method according to claim 3, characterised in that it comprises dissolving the p-butoxyphenylacetylhydroxamic acid in a solution of an alkaline base metal.

5. A method according to any one of claims 1 to 4, characterised in that a solution of salifying agent is used which is slightly in excess in relation to the quantity of p-butoxyphenylacetylhydroxamic acid to be dissolved therein.

6. A method according to any one of claims 1 to 5, characterised in that for the preparation of the p-butoxyphenylacetylhydroxamic acid there is used a solution of an acid chosen from the group of tartaric, aspartic, citric, phosphoric, sulphuric and hydrochloric acids.

7. A method according to any one of claims 1 to 6, characterised in that it comprises precipitating the p-butoxyphenylacetylhydroxamic acid by adding a solution of sodium salt in a solution of hydrochloric acid adjusted to ambient temperature and containing a polyoxyethylenated ester of sorbitol.

8. A method according to any one of claims 1 to 7, characterised in that it comprises precipitating the p-butoxyphenylacetylhydroxamic acid in the presence of suitable excipients, without isolating the precipitated p-butoxyphenylacetylhydroxamic acid.